# EUROPÄISCHE PATENTSCHRIFT

(11) **EP 0 835 249 B1**
(45) Veröffentlichungstag und Bekanntmachung des Hinweises auf die Patenterteilung: **29.08.2001**
(21) Anmeldenummer: 96920832.1
(22) Anmeldetag: 20.06.1996
(51) Int. Cl.: C07D 249/08, C07D 249/12, C07D 249/10, A01N 43/653, A01N 47/24, A01N 47/30

(54) **IMINOOXYMETHYLENANILIDE UND IHRE VERWENDUNG ZUR BEKÄMPFUNG VON TIERISCHEN SCHÄDLINGEN UND SCHADPILZEN**
IMINOOXYMETHYLENE ANILIDES AND THEIR USE AS PESTICIDES AND FUNGICIDES
IMINOOXYMETHYLENANILIDES ET LEUR UTILISATION DANS LA LUTTE CONTRE LES ANIMAUX ET CHAMPIGNONS NUISIBLES

(30) Priorität: 27.06.1995 DE 19523289
(43) Veröffentlichungstag der Anmeldung: 15.04.1998
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); RÖHL, Franz, D-67105 Schifferstadt (DE); GÖTZ, Norbert, D-67547 Worms (DE); LORENZ, Gisela, D-67434 Hambach (DE); AMMERMANN, Eberhard, D-64646 Heppenheim (DE); STRATHMANN, Siegfried, D-67117 Limburgerhof (DE)
(74) Vertreter: Münch, Volker, Dr.
(86) Internationale Anmeldenummer: EP9602664
(87) Internationale Veröffentlichungsnummer: WO9701544

(56) Entgegenhaltungen:
- WO-A-93/15046
- WO-A-96/16044
- DE-A- 4 438 824
- US-A- 4 865 837
- CHEMICAL PHARMACEUTICAL BULLETIN, Bd. 41, Nr. 7, Juli 1993, TOKYO JP, Seiten 1226-31, XP002012322 S. OHTA ET AL.: "Synthesis and application of triazole derivatives. Synthesis of 3- and 5-acyl-1,2,4-triazoles via lithiation of 1-alkyl-1H-1,2,4-triazoles"
- HELVETICA CHIMICA ACTA, Bd. 60, Nr. 7, 2.November 1977, BASEL CH, Seiten 2171-6, XP002012323 M.R.H. ELMOGHAYAR ET AL.: "Reactions with carbo(heterylhydrazonoyl) halides. I. Chemistry of carbo(3-phenylpyrazol-5-yl-hydrazonoyl) chlorides"
- CHEMISCHE BERICHTE, Bd. 96, Nr. 12, 6.Dezember 1963, WEINHEIM DE, Seiten 3120-32, XP002012324 M. REGITZ ET AL.: "Über die Bildung von 1,2,4-Triazolderivaten aus alpha-acetamino-beta- dicarbonylverbindungen durch Japp-Klingemann-Spaltung mit Diazoniumsalzen"
- JOURNAL OF THE CHEMICAL SOCIETY, Februar 1962, LONDON GB, Seiten 575-83, XP002012325 E.J. BROWNE ET AL.: "Triazoles. Part VI. 1,5-Diaryl-1,2,4-triazole-3-aldehydes"
- JOURNAL OF THE CHEMICAL SOCIETY, PERKIN TRANSACTIONS 1, 1977, LONDON GB, Seiten 589-91, XP002012326 M. RUCCIA ET AL.: "Mononuclear heterocyclic rearrangements. Part 10. Rearrangements in the 1,2,5-oxadiazole series"
- DATABASE CROSSFIRE Beilstein Informationssysteme GmbH, Frankfurt DE XP002012327 & HETEROCYCLES, Bd. 15, Nr. 1, 1981, Seiten 301-4,
- DATABASE CROSSFIRE Beistein Informationssysteme GmbH, Frankfurt DE XP002012328 & HETEROCYCLES, Bd. 4, 1976, Seite 1655, 1657
- DATABASE CROSSFIRE Beilstein Informationssysteme GmbH, Frankfurt DE XP002012329 & HETEROCYCLES, Bd. 31, Nr. 5, 1990, Seiten 869-75,
- DATABASE CROSSFIRE Beistein Informationssysteme GmbH, Frankfurt DE XP002012330 & CHEM. BER., Bd. 26, 1893, Seite 2786
- DATABASE CROSSFIRE Beisltein Infotmationssysteme GmbH, Frankfurt DE XP002012331 & JUSTUS LIEBIGS ANN. CHEM., Bd. 526, 1936, Seite 173, 179
- DATABASE CROSSFIRE Beistein Informationssysteme GmbH, Frankfurt DE XP002012332 & CHIMIJA CHIM. TECHNOL. (NDVS), Nr. 2, 1959, Seite 341, 342, 344

## Beschreibung

Die vorliegende Erfindung betrifft Iminooxymethylenanilide der Formel I 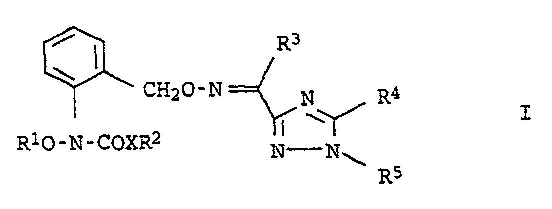 in der der Index und die Substituenten die folgende Bedeutung haben:
- X: O;
- R¹: Wasserstoff, C₁-C₄-Alkyl, Allyl, Propargyl oder Methoxymethyl;
- R²: C₁-C₄-Alkyl, Cyclopropyl oder Trifluormethyl;
- R³: Cyano, Halogen, C₁-C₄-Alkyl, Trifluormethyl, Cyclopropyl, Methoxy, Ethoxy und Methylthio;
- R⁴: Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio;
Phenyl oder Hetaryl, wobei diese Reste einen bis drei der folgenden Gruppen tragen kann: Cyano, Halogen, C₁-C₄-Alkyl und CRⁱⁱⁱ=NOR^{iv};
- R⁵: Phenyl, welches ein bis drei der folgenden Reste tragen kann: Cyano, Halogen, C₁-C₄-Alkyl oder CRⁱⁱⁱ=NOR^{iv}; oder welches durch C₁-C₆-Alkyl, C₂-C₆-Alkenyl, C₂-C₆-Alkinyl, C₁-C₅-Halogenalkyl, C₂-C₆-Halogenalkenyl, C₂-C₆-Halogenalkinyl, C₁-C₆-Alkoxy, C₂-C₆-Alkenyloxy, C₂-C₆-Alkinyloxy, C₁-C₆-Halogenalkyloxy, C₂-C₆-Halogenalkenyloxy, C₂-C₆-Halogenalkinyloxy, Phenyl, Phenoxy, Hetaryl oder Hetaryloxy substituiert ist, wobei die Phenyl- und Hetaryl-Gruppen ihrerseits durch Cyano, Halogen, C₁-C₄-Alkyl und CRⁱⁱⁱ=NOR^{iv} substituiert sein können;
- Rⁱⁱⁱ: Wasserstoff, C₁-C₆-Alkyl, C₂-C₆-Alkenyl oder C₂-C₆-Alkinyl;
- R^{iv}: C₁-C₆-Alkyl, C₂-C₆-Alkenyl, C₂-C₆-Alkinyl oder Phenyl-C₁-C₆-alkyl;
- Hetaryl: 5-Ring Heteroaromat enthaltend ein bis drei Stickstoffatome und/oder ein Sauerstoff- oder Schwefelatom; oder 6-Ring Heteroaromaten enthaltend ein bis vier Stickstoffatome.

Außerdem betrifft die Erfindung Verfahren und Zwischenprodukte zur Herstellung dieser Verbindungen sowie sie enthaltende Mittel und deren Verwendung zur Bekämpfung von tierischen Schädlingen und Schadpilzen.

In der Literatur sind Anilide mit fungizider Wirksamkeit beschrieben (WO-A 93/15,046). Außerdem werden in der WO-A 96/16044 Hetaryliminooxymethylenanilide in allgemeiner Form beschrieben.

Der vorliegenden Erfindung lagen demgegenüber Verbindungen mit verbesserter Wirksamkeit als Aufgabe zugrunde.

Demgemäß wurden die eingangs definierten Verbindungen I gefunden. Außerdem wurden Verfahren und Zwischenprodukte zur Herstellung dieser Verbindungen sowie sie enthaltende Mittel und deren Verwendung zur Bekämpfung von tierischen Schädlingen und Schadpilzen gefunden.

Die Verbindungen I sind auf verschiedenen Wegen herstellbar. Besonders vorteilhaft erhält man diejenigen Verbindungen I, in denen R¹ nicht Wasserstoff bedeutet, dadurch, daß man ein Methylenanilid der Formel II mit einem Oxim der Formel III oder dessen Salz umsetzt.

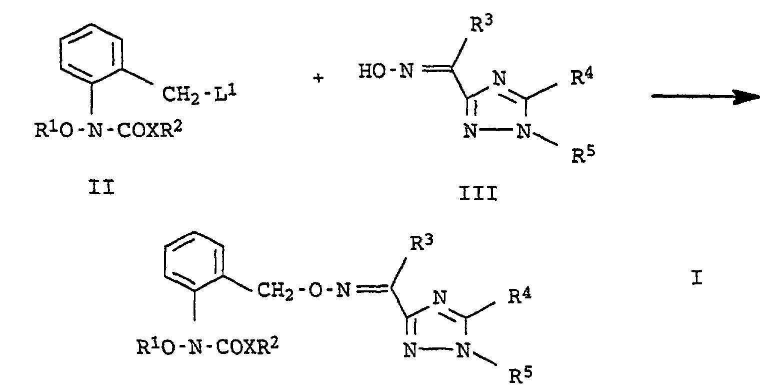

L¹ in der Formel II steht für eine Abgangsgruppe, d.h. eine nucleophil austauschbare Gruppe wie Halogen (z.B. Chlor, Brom und Iod), oder ein Alkyl- oder Arylsulfonat (z.B. Methylsulfonat, Trifluormethylsulfonat, Phenylsulfonat und 4-Methylsulfonat).
Die Oxime III können auch in Form ihrer Salze, z.B. mit anorganischen Säuren wie Hydrochloride, Hydrobromide, Hydrosulfate und Hydrophosponate, verwendet werden.

Die Umsetzung der Verbindungen II und III erfolgt üblicherweise bei Temperaturen von 0°C bis 80°C, vorzugsweise 20°C bis 60°C, in einem inerten Lösungsmittel in Gegenwart einer Base.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Dieethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, tert.-Butylmethylether und Dimethylform-amid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calciumhydroxid), Alkalimetall- und Erdalkalimetalloxide (z.B. Lithiumoxid, Natriumoxid, Calciumoxid und Magnesiumoxid), Alkalimetall- und Erdalkalimetallhydride (z.B. Lithiumhydrid, Natriumhydrid, Kaliumhydrid und Calciumhydrid), Alkalimetallamide (z.B. Lithiumamid, Natriumamid und Kaliumamid), Alkalimetall- und Erdalkalimetallcarbonate (z.B. Lithiumcarbonat und Calciumcarbonat) sowie Alkalimetallhydrogencarbonate (z.B. Natriumhydrogencarbonat), metallorganische Verbindungen, insbesondere Alkalimetallalkyle (z.B. Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden, Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat) sowie Phosphoniumhalogenide (z.B. Tetrabutylphosphoniumchlorid und Tetraphenylphosphoniumbromid) in Betracht.

Die für die Herstellung der Verbindungen I benötigten Ausgangsstoffe II sind aus WO-A 93/15,046 bekannt oder können nach den dort beschriebenen Methoden hergestellt werden.

Die Oxime der Formel III erhält man vorteilhaft dadurch, daß man eine Verbindung VI
in einem inerten Lösungsmittel mit ggf. einer Carbonylverbindung VIIa bzw. VIIb in das entsprechende Triazolylketon V überführt und V anschließend mit Hydroxylamin (HO-NH₂) oder dessen Salz umsetzt.

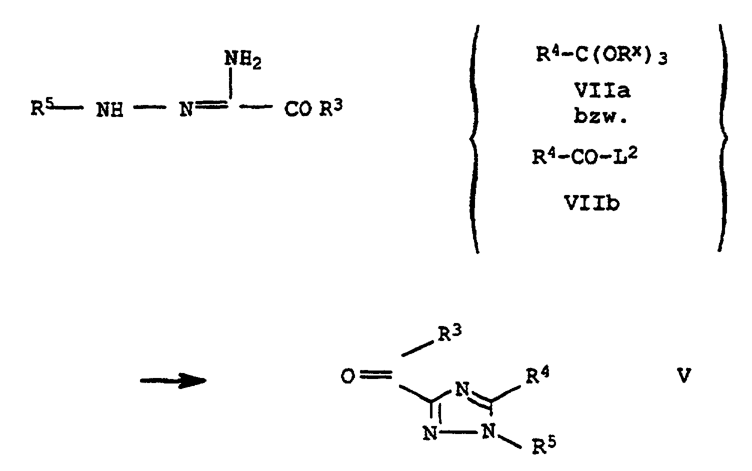

In der Formel VIIa bedeutet R^{x} eine Alkyl-, Alkenyl- oder Alkinylgruppe mit jeweils bis zu 6 Kohlenstoffatomen (besonders C₁-C₄-Alkyl, C₃-C₆-Alkenyl oder C₃-C₆-Alkinyl). In der formel VIIb bedeutet L² eine Abgangsgruppe, d.h. eine nucleophil austauschbare Gruppe wie Halogen (z.B. Chlor, Brom und Iod), oder ein Alkyloder Arylsulfonat (z.B. Methylsulfonat, Trifluormethylsulfonat, Phenylsulfonat und 4-Methylsulfonat).

Die Verbindungen VI sind literaturbekannt oder können ausgehend von Verbindungen des Typs R⁵NHN=C(Cl)COR³ nach literaturbekannten Methoden hergestellt werden (DE-A 27 50 813; Chem. Ber. 26, 2783; Chem. Ber. 25, 3541; J. Prakt. Chem. 64, 230).

Die Herstellung der Verbindungen des Typs R⁵NHN=C(Cl)COR³ ist beispielsweise in J. Chem. Res., Syn. 1993, 358; Zh. Oschch. Khim. 63, 825 (1993), Sulfur Lett. 15, 103 (1992); Pharmazie 43, 832 (1988); Heterocycles 26, 1283 (1987); Heterocycles 32, 1879 (1991); Chem. Ber. 38, 2989 (1905); Bull. Soc. Chim. Fr. 41, 1495 (1927); Bull. Soc. Chim. Fr. 41, 1605 (1927); DE-A 21 56 058; J. Org. Chem. 37, 386 (1972); DE-A 20 17 762; J. Org. Khim. USSR 4, 634 (1968) beschrieben.

Die Umsetzung von VI mit VIIa bzw. VIIb erfolgt üblicherweise bei Temperaturen von 0°C bis 150°C, vorzugsweise 20°C bis 120°C ohne Lösungsmittel oder in einerm inerten Lösungsmittel.
Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid, Dimethylformamid, N-Methylpyrrolidon oder Essigsäureethylester, besonders bevorzugt Toluol, tert.-Butylmethylether, Ethanol oder Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natronlauge, Kaliumcarbonat, Pyridin und Triethylamin.

Die Basen werden im allgemeinen in katalytischen Mengen eingesetzt, sie können aber auch äquimolar, im Überschuß oder gegebenenfalls als Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure, p-Toluolsulfonsäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, VIIa bzw. VIIb in einem Über- oder Unterschuß bezogen auf VI einzusetzen.

Die Umsetzung von V mit Hydroxylamin erfolgt üblicherweise bei Temperaturen von 0°C bis 100°C, vorzugsweise 20°C bis 50°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchiorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, N-Methylpyrrilidon und Essigsäureethylester, besonders bevorzugt Wasser, Methanol, Ethanol, Toluol und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natronlauge, Kaliumcarbonat, Pyridin und Triethylamin.

Die Basen werden im allgemeinen in katalytischen Mengen eingesetzt, sie können aber auch äquimolar, im Überschuß oder gegebenenfalls als Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propion-säure, Oxalsäure, Zitronensäure, p-Toluolsulfonsäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, Hydroxylamin in einem Über- oder Unterschuß bezogen auf V einzusetzen.

In entsprechender Weise erhält man die Verbindungen I auch dadurch, daß man einen Hydroxylaminether der Formel IV in einem inerten organischen Lösungsmittel mit einem Triazolylketon der Formel V umsetzt.

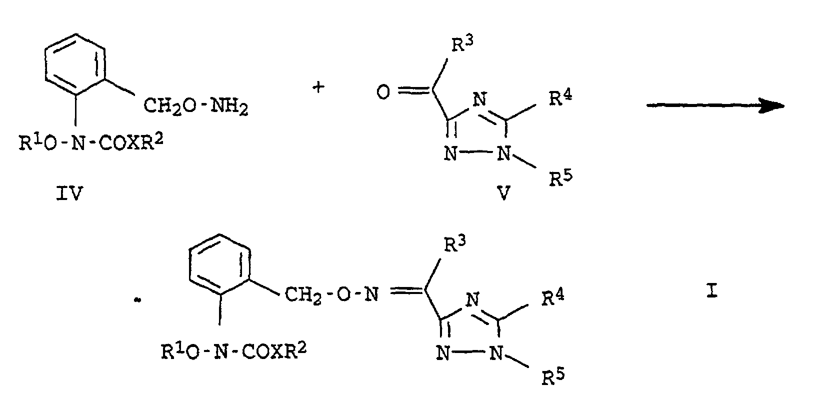

Diese Umsetzung verläuft im allgemeinen und im besonderen unter den für die Umsetzung von II mit III beschriebenen Bedingungen.

Außerdem kann die Umsetzung des Benzyloxyamins IV mit den Carbonylverbindungen V auch unter neutralen oder sauren Bedingungen durchgeführt werden.

A1s saure Katalysatoren kommen Mineralsäure wie z.B. Salzsäure, Schwefelsäure, Phosphorsäure oder Salpetersäure, oder auch organische Säuren wie z.B. Ameisensäure, Essigsäure, Propionsäure, Triethylamin-hydrochlorid, p-Toluolsulfonsäure, Methansulfonsäure, Citronensäure oder saurer Ionenaustauscher in Betracht.

Nach einem weiteren Verfahren erhält man die Verbindungen I beispielsweise dadurch, daß man eine Benzylverbindung IIa analog dem vorstehend beschriebenen Verfahren mit einem Oxim der Formel III zum Nitrobenzol IXa umsetzt, IXa zum N-Hydroxyanilid IXb reduziert und IXb gemäß dem folgenden Reaktionsschema in an sich bekannter Weise zu I umsetzt.

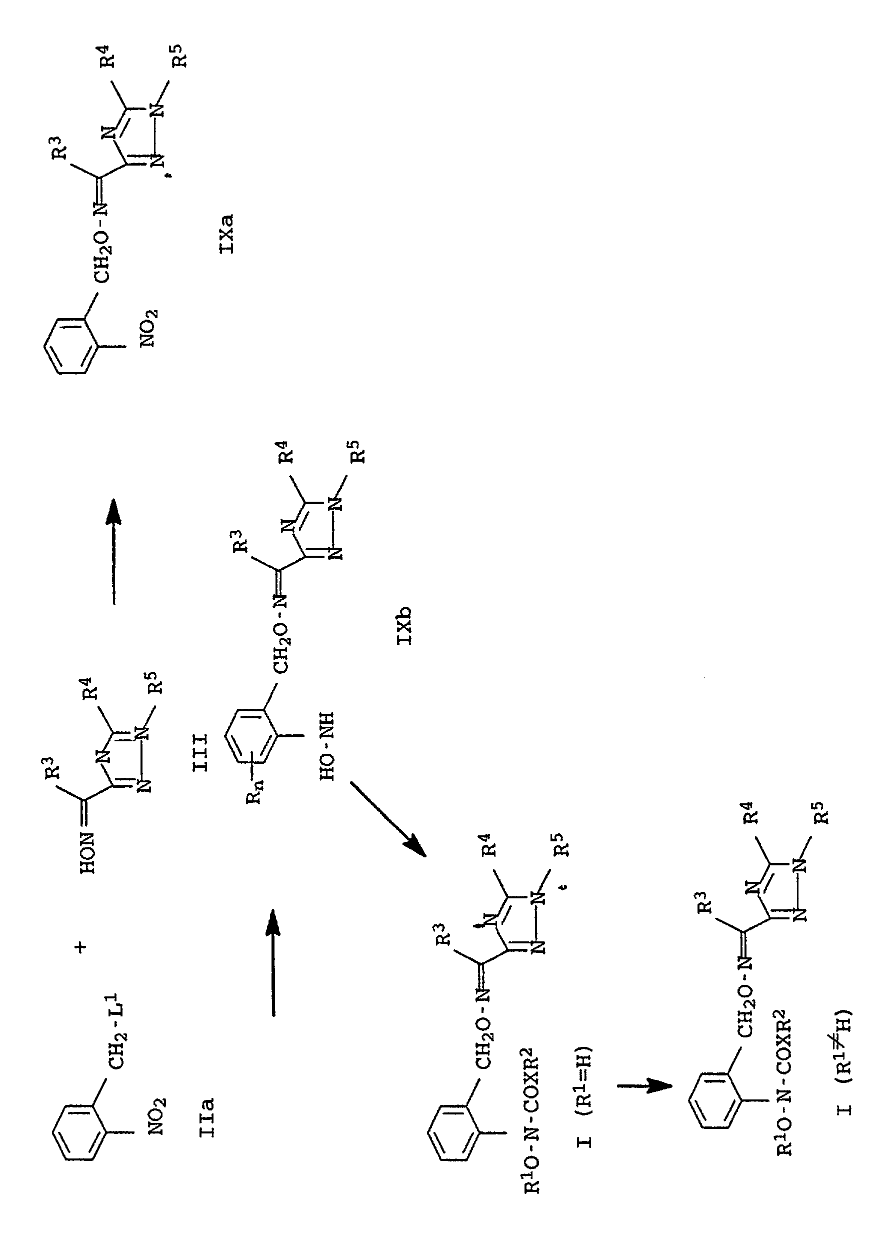

Die Umwandlung der Nitrogruppe in die Gruppierung N(OR¹)COXR² erfolgt im allgemeinen und im besonderen nach den in WO-A93/15,046, DE Pat. Anm. P 44 23 612.3, DE Pat. Anm. P 44 23 613.1, DE Pat. Anm. P 44 41 673.3, DE Pat. Anm. P 44 41 674.1, DE Pat. Anm. P 44 41 676.8 und DE Pat. Anm. P 19 502 700.0 beschrioebenen Verfahren.

Säuren für Säureadditionsprodukte der Verbindungen I sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure) oder andere protonenacide Verbindugnen (z.B. Saccharin).

Die Verbindungen I können in Bezug auf die C=N-Doppelbindung sowohl in der E- als auch in der Z-Konfiguration vorliegen. Beide Isomere können erfindungsgemäß gemeinsam oder getrennt angewendet werden. Im Hinblick auf die Wirkung gegen Schadpilze ist insbesondere dasjenige Isomer bevorzugt, in dem der Hydroxylamin-Sauerstoff und der Triazolylrest trans ständig sind.

Bei den in den vorstehenden Formeln angegebenen Definitionen der Symbole wurden z.T. Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 6 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 6 Kohlenstoffatomen (wie vorstehend genannt), welche über ein Sauerstoffatom (-O-) an das Gerüst gebunden sind; Halogenalkoxy: geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
Alkenyl: ungesättigte, geradkettige oder verzweigte Kohlenwasserstoffreste mit 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkenyloxy: geradkettige oder verzweigte Alkenylgruppen mit vorzugsweise 3 bis 6 Kohlenstoffatomen (wie vorstehend genannt), welche über ein Sauerstoffatom (-O-) an das Gerüst gebunden sind;
Alkinyl: geradkettige oder verzweigte Kohlenwasserstoffgruppen mit 2 bis 6 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-l-methyl-2-propinyl;
Alkinyloxy: geradkettige oder verzweigte Alkinylgruppen mit vorzugsweise 3 bis 6 Kohlenstoffatomen (wie vorstehend genannt), welche über ein Sauerstoffatom (-O-) an das Gerüst gebunden sind;
Cycloalkyl: mono- oder bicyclische Kohlenwaserstoffreste mit vorzugsweise 3 bis 10 Kohlenstoffatomen, z.B. C₃-C₁₀-(Bi)cycloalkyl wie Cyclopropyl, Cyclobutyl, Cyclopentyl, Cyclohexyl, Cycloheptyl, Bornanyl, Norbornanyl, Dicyclohexyl, Bicyclo[3,3,0]octyl, Bicyclo [3,2,1] octyl, Bicyclo[2,2,2]octyl oder Bicyclo[3,3,1]nonyl;
lin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl,
insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring-Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz *"ggf. subst"* in bezug auf die aromatischen Gruppen soll zum Ausdruck bringen, daß diese Gruppen einen bis drei der folgenden Reste:
Cyano, Halogen, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 6, insbesondere 2 bis 4 Kohlenstoffatome enthalten;
oder einen der folgenden Reste
unsubstituiertes oder substituiertes Phenyl, Phenoxy, Hetaryl, Hetaryloxy, wobei die Hetarylreste 5 oder 6 Ringglieder enthalten;
oder einen Rest:
CRⁱⁱⁱNOR^{iv}, wobei Rⁱⁱⁱ Wasserstoff, Alkyl, Alkenyl oder Alkinyl und R^{iv} Alkyl, Alkenyl, Alkinyl und Phenylalkyl bedeutet und wobei die genannten Alkylgruppen vorzugsweise 1 bis 6 Kohlenstoffatome, insbesondere 1 bis 4 Kohlenstoffatome, und die Alkenyl- bzw. Alkinylgruppen vorzugsweise 2 bis 6 Kohlenstoffatome enthalten und Phenyl unsubstituiert ist oder durch übliche Gruppen substituiert sein kann,
tragen können.

Daneben werden Verbindungen I bevorzugt, in denen R¹ für C₁-C₄-Alkyl, insbesondere Methyl, steht.

Außerdem werden Verbindungen I besonders bevorzugt, in denen R¹ für Wasserstoff steht.

Gleichermaßen besonders bevorzugt sind Verbindungen I, in denen R¹ für Allyl, Propargyl und Methoxymethyl steht.

Daneben werden Verbindungen I bevorzugt, in denen R² für C₁-C₄-Alkyl, insbesondere Methyl steht.

Außerdem werden Verbindungen I besonders bevorzugt, in denen R² für Cyclopropyl steht.

Gleichermaßen besonders bevorzugt sind Verbindungen I, in denen R² für Trifluormethyl steht.

Daneben werden Verbindungen I bevorzugt, in denen R³ für C₁-C₄-Alkyl, insbesondere Methyl steht.

Außerdem werden Verbindungen I besonders bevorzugt, in denen R³ für Cyclopropyl steht.

Gleichermaßen besonders bevorzugt sind Verbindungen I, in denen R³ für Cyano, Trifluormethyl, Halogen, Methoxy, Ethoxy und Methylthio steht.

Daneben werden Verbindungen I bevorzugt, in denen R⁴ für Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio steht.

Außerdem werden Verbindungen I bevorzugt, in denen R⁴ für ggf. subst. Phenyl oder Hetaryl steht.

Daneben werden Verbindungen I bevorzugt, in denen die Gruppe R⁴ ein bis drei der folgenden Gruppen trägt: Cyano, Halogen, C₁-C₄-Alkyl und CRⁱⁱⁱ=NOR^{iv}.

Außderdem werden Verbindungen I bevorzugt, in denen R⁵ für ggf. substituiertes Phenyl steht.

Außerdem werden Verbindungen I bevorzugt, in denen R⁵ ein bis drei der folgenden Gruppen trägt: Cyano, Halogen, C₁-C₄-Alkyl und CRⁱⁱⁱ=NOR^{iv}.

Desweiteren werden Verbindugnen I bevorzugt, in denen R⁵ durch C₁-C₆-Alkyl, C₂-C₆-Alkenyl, C₂-C₆-Alkinyl, C₁-C₆-Halogenalkyl, C₂-C₆-Halogenalkenyl und C₂-C₆-Halogenalkinyl substituiert ist. Gleichermaßen besonders bevorzugt sind Verbindungen I, in denen R⁵ durch C₁-C₆-Alkoxy, C₂-C₆-Alkenyloxy, C₂-C₆-Alkinyloxy, C₁-C₆-Halogenalkyloxy, C₂-C₆-Halogenalkenyloxy und C₂-C₆-Halogenalkinyloxy substituiert ist.

Daneben werden Verbindungen I besonders bevorzugt, in denen R⁵ durch Phenyl, Phenoxy, Hetaryl oder Hetaryloxy substituiert ist, wobei diese Gruppen ihrerseits durch übliche Gruppen substituiert sein können.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der Formel I.A, in der R¹ Wasserstoff, XR² Methoxy, R³ Methyl und R⁴ Wasserstoff bedeutet und R^{x} für eine Verbindung jeweils einer Zeile der Tabelle A entspricht

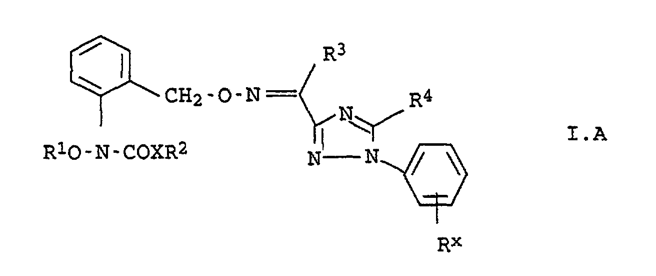

### Tabelle 2

Verbindungen der Formel I.A, in der R¹ Wasserstoff, XR² Methoxy, R³ Methyl und R⁴ Methyl bedeutet und R^{x} für eine Verbindung jeweils einer Zeile der Tabelle A entspricht

### Tabelle 3

Verbindungen der Formel I.A, in der R¹ Wasserstoff, XR² Methoxy, R³ Methyl und R⁴ Trifluormethyl bedeutet und R^{x} für eine Verbindung jeweils einer Zeile der Tabelle A entspricht

### Tabelle 4

Verbindungen der Formel I.A, in der R¹ Wasserstoff, XR² Methoxy, R³ Methyl und R⁴ Methoxy bedeutet und R^{x} für eine Verbindung jeweils einer Zeile der Tabelle A entspricht

### Tabelle 5

Verbindungen der Formel I.A, in der R¹ Wasserstoff, XR² Methoxy, R³ Trifluormethyl und R⁴ Wasserstoff bedeutet und R^{x} für eine Verbindung jeweils einer Zeile der Tabelle A entspricht

### Tabelle 6

Verbindungen der Formel I.A, in der R¹ Wasserstoff, XR² Methoxy, R³ Trifluormethyl und R⁴ Methyl bedeutet und R^{x} für eine Verbindung jeweils einer Zeile der Tabelle A entspricht

### Tabelle 7

Verbindungen der Formel I.A, in der R¹ Wasserstoff, XR² Methoxy, R³ Trifluormethyl und R⁴ Trifluormethyl bedeutet und R^{x} für eine Verbindung jeweils einer Zeile der Tabelle A entspricht

### Tabelle 8

Verbindungen der Formel I.A, in der R¹ Wasserstoff, XR² Methoxy, R³ Trifluormethyl und R⁴ Methoxy bedeutet und R^{x} für eine Verbindung jeweils einer Zeile der Tabelle A entspricht

### Tabelle 9

Verbindungen der Formel I.A, in der R¹ Methyl, XR² Methoxy, R³ Methyl und R⁴ Wasserstoff bedeutet und R^{x} für eine Verbindung jeweils einer Zeile der Tabelle A entspricht

### Tabelle 10

Verbindungen der Formel I.A, in der R¹ Methyl, XR² Methoxy, R³ Methyl und R⁴ Methyl bedeutet und R^{x} für eine Verbindung jeweils einer Zeile der Tabelle A entspricht

### Tabelle 11

Verbindungen der Formel I.A, in der R¹ Methyl, XR² Methoxy, R³ Methyl und R⁴ Trifluormethyl bedeutet und R^{x} für eine Verbindung jeweils einer Zeile der Tabelle A entspricht

### Tabelle 12

Verbindungen der Formel I.A, in der R¹ Methyl, XR² Methoxy, R³ Methyl und R⁴ Methoxy bedeutet und R^{x} für eine Verbindung jeweils einer Zeile der Tabelle A entspricht

### Tabelle 13

Verbindungen der Formel I.A, in der R¹ Methyl, XR² Methoxy, R³ Trifluormethyl und R⁴ Wasserstoff bedeutet und R^{x} für eine Verbindung jeweils einer Zeile der Tabelle A entspricht

### Tabelle 14

Verbindungen der Formel I.A, in der R¹ Methyl, XR² Methoxy, R³ Trifluormethyl und R⁴ Methyl bedeutet und R^{x} für eine Verbindung jeweils eines Zeile der Tabelle A entspricht

### Tabelle 15

Verbindungen der Formel I.A, in der R¹ Methyl, XR² Methoxy, R³ Trifluormethyl und R⁴ Trifluormethyl bedeutet und R^{x} für eine Verbindung jeweils einer Zeile der Tabelle A entspricht

### Tabelle 16

Verbindungen der Formel I.A, in der R¹ Methyl, XR² Methoxy, R³ Trifluormethyl und R⁴ Methoxy bedeutet und R^{x} für eine Verbindung jeweils einer Zeile der Tabelle A entspricht

### Tabelle 17

Verbindungen der Formel I.A, in der R¹ Wasserstoff, XR² Methoxy, R³ Methyl und R⁴ Ethoxy bedeutet und R^{x} für eine Verbindung jeweils einer Zeile der Tabelle A entspricht

**Tabelle A**

| Nr. | R^{x} |
|---|---|
| 1 | H |
| 2 | 2-F |
| 3 | 3-F |
| 4 | 4-F |
| 5 | 2,4-F₂ |
| 6 | 2,4,6-F₃ |
| 8 | 2,3-F₂ |
| 9 | 2-Cl |
| 10 | 3-Cl |
| 11 | 4-Cl |
| 12 | 2,3-Cl₂ |
| 13 | 2,4-Cl₂ |
| 14 | 2,5-Cl₂ |
| 15 | 2,6-Cl₂ |
| 16 | 3,4-Cl₂ |
| 17 | 3,5-Cl₂ |
| 18 | 2,3,4-Cl₃ |
| 19 | 2,3,5-Cl₃ |
| 20 | 2,3,6-Cl₃ |
| 21 | 2,4,5-Cl₃ |
| 22 | 2,4,6-Cl₃ |
| 23 | 3,4,5-Cl₃ |
| 39 | 2-Br |
| 40 | 3-Br |
| 41 | 4-Br |
| 42 | 2,4-Br₂ |
| 43 | 2,5-Br₂ |
| 44 | 2,6-Br₂ |
| 45 | 2,4,6-Br₃ |
| 47 | 2-J |
| 48 | 3-J |
| 49 | 4-J |
| 50 | 2,4-J₂ |
| 51 | 2-Cl, 3-F |
| 52 | 2-C1, 4-F |
| 53 | 2-Cl, 5-F |
| 54 | 2-Cl, 6-F |
| 55 | 2-Cl, 3-Br |
| 56 | 2-C1, 4-Br |
| 57 | 2-Cl, 5-Br |
| 58 | 2-Cl, 6-Br |
| 59 | 2-Br, 3-Cl |
| 60 | 2-Br, 4-C1 |
| 61 | 2-Br, 5-Cl |
| 62 | 2-Br, 3-F |
| 63 | 2-Br, 4-F |
| 64 | 2-Br, 5-F |
| 65 | 2-Br, 6-F |
| 66 | 2-F, 3-Cl |
| 67 | 2-F, 4-Cl |
| 68 | 2-F, 5-C1 |
| 69 | 3-Cl, 4-F |
| 70 | 3-Cl, 5-F |
| 71 | 3-Cl, 4-Br |
| 72 | 3-Cl, 5-Br |
| 73 | 3-F, 4-Cl |
| 74 | 3-F, 4-Br |
| 75 | 3-Br, 4-Cl |
| 76 | 3-Br, 4-F |
| 77 | 2,6-Cl₂, 4-Br |
| 78 | 2-CH₃ |
| 79 | 3-CH₃ |
| 80 | 4-CH₃ |
| 81 | 2,3-(CH₃)₂ |
| 82 | 2,4-(CH₃)₂ |
| 83 | 2,5-(CH₃)₂ |
| 84 | 2,6-(CH₃)₂ |
| 85 | 3,4-(CH₃)₂ |
| 86 | 3,5-(CH₃)₂ |
| 87 | 2,3,5-(CH₃)₃ |
| 88 | 2,3,4-(CH₃)₃ |
| 89 | 2,3,6-(CH₃)₃ |
| 90 | 2,4,5-(CH₃)₃ |
| 91 | 2,4,6-(CH₃)₃ |
| 92 | 3,4,5-(CH₃)₃ |
| 96 | 2-C₂H₅ |
| 97 | 3-C₂H₅ |
| 98 | 4-C₂H₅ |
| 99 | 2,4-(C₂H₅)₅ |
| 100 | 2,6-(C₂H₅)₂ |
| 101 | 3,5-(C₂H₅)₂ |
| 102 | 2,4,6-(C₂H₅)₃ |
| 103 | 2-n-C₃H₇ |
| 104 | 3-n-C₃H₇ |
| 105 | 4-n-C₃H₇ |
| 106 | 2-i-C₃H₇ |
| 107 | 3-i-C₃H₇ |
| 108 | 4-i-C₃H₇ |
| 109 | 2,4-(i-C₃H₇)₂ |
| 110 | 2,6-(i-C₃H₇)₂ |
| 111 | 3,5-(i-C₃H₇)₂ |
| 112 | 2-s-C₄H₉ |
| 113 | 3-s-C₄H₉ |
| 114 | 4-s-C₄H₉ |
| 115 | 2-t-C₄H₉ |
| 116 | 3-t-C₄H₉ |
| 117 | 4-t-C₄H₉ |
| 118 | 4-n-C₉H₁₉ |
| 119 | 2-CH₃, 4-t-C₄H₉ |
| 120 | 2-CH₃, 6-t-C₄H₉ |
| 121 | 2-CH₃, 4-i-C₃H₇ |
| 122 | 2-CH₃, 5-i-C₃H₇ |
| 123 | 3-CH₃, 4-i-C₃H₇ |
| 124 | 2-Cyclo-C₆H₁₁ |
| 125 | 3-cyclo-C₆H₁₁ |
| 126 | 4-cyclo-C₆H₁₁ |
| 127 | 2-C1, 4-C₆H₅ |
| 128 | 2-Br, 4-C₆H₅ |
| 129 | 2-OCH₃ |
| 130 | 3-OCH₃ |
| 131 | 4-OCH₃ |
| 132 | 2-OC₂H₅ |
| 133 | 3-O-C₂H₅ |
| 134 | 4-O-C₂H₅ |
| 135 | 2-O-n-C₃H₇ |
| 136 | 3-O-n-C₃H₇ |
| 137 | 4-O-n-C₃H₇ |
| 138 | 2-O-i-C₃H₇ |
| 139 | 3-O-i-C₃H₇ |
| 140 | 4-O-i-C₃H₇ |
| 141 | 2-O-n-C₆H₁₃ |
| 142 | 3-O-n-C₆H₁₃ |
| 143 | 4-O-n-C₆H₁₃ |
| 144 | 2-O-CH₂C₆H₅ |
| 145 | 3-O-CH₂C₆H₅ |
| 146 | 4-O-CH₂C₆H₅ |
| 147 | 2-O-(CH₂)₃C₆H₅ |
| 148 | 4-O-(CH₂)₃C₆H₅ |
| 155 | 2-O-t-C₄H₉ |
| 156 | 3-O-t-C₄H₉ |
| 157 | 4-O-t-C₄H₉ |
| 158 | 3-(3'-Cl-C₆H₄) |
| 159 | 4-(4'-CH₃-C₆H₄) |
| 160 | 2-O-C₆H₅ |
| 161 | 3-O-C₆H₅ |
| 162 | 4-O-C₆H₅ |
| 163 | 2-O-(2'-F-C₆H₄) |
| 164 | 3-O-(3'-Cl-C₆H₄) |
| 165 | 4-O-(4'-CH₃-C₆H₄) |
| 166 | 2,3,6-(CH₃)₃, 4-F |
| 167 | 2,3,6-(CH₃)₃, 4-Cl |
| 168 | 2,3,6-(CH₃)₃, 4-Br |
| 169 | 2,4-(CH₃)₂, 6-F |
| 170 | 2,4-(CH₃)₂, 6-Cl |
| 171 | 2,4-(CH₃)₂, 6-Br |
| 172 | 2-i-C₃H₇, 4-Cl, 5-CH₃ |
| 181 | 2-CH₃, 5-i-C₃H₇, 4-Cl |
| 188 | 2-Me-4-CH₃-CH(CH₃)-CO |
| 189 | 2-CH₃-4-(CH₃-C=NOCH₃) |
| 190 | 2-CH₃-4-(CH₃-C=NOC₂H₅) |
| 191 | 2-CH₃-4-(CH₃-C=NO-n-C₃H₇) |
| 192 | 2-CH₃-4-(CH₃-C=NO-i-C₃H₇) |
| 193 | 2,5- (CH₃)₂-4-(CH₃-C=NOCH₃) |
| 194 | 2,5-(CH₃)₂-4-(CH₃-C=NOC₂H₅) |
| 195 | 2,5-(CH₃-4-(CH₃-C=NO-n-C₃H₇) |
| 196 | 2,5-(CH₃)₂-4-(CH₃-C=NO-i-C₃H₇) |
| 197 | 2-C₆H₅ |
| 198 | 3-C₆H₅ |
| 199 | 4-C₆H₅ |
| 200 | 2-(2'-F-C₆H₄) |
| 201 | 2-CH₃, 5-Br |
| 202 | 2-CH₃, 6-Br |
| 203 | 2-Cl, 3-CH₃ |
| 204 | 2-Cl, 4-CH₃ |
| 205 | 2-Cl, 5-CH₃ |
| 206 | 2-F, 3-CH₃ |
| 207 | 2-F, 4-CH₃ |
| 208 | 2-F, 5-CH₃ |
| 209 | 2-Br, 3-CH₃ |
| 210 | 2-Br, 4-CH₃ |
| 211 | 2-Br, 5-CH₃ |
| 212 | 3-CH₃, 4-Cl |
| 213 | 3-CH₃, 5-Cl |
| 214 | 3-CH₃, 4-F |
| 215 | 3-CH₃, 5-F |
| 216 | 3-CH₃, 4-Br |
| 217 | 3-CH₃, 5-Br |
| 218 | 3-F, 4-CH₃ |
| 219 | 3-Cl, 4-CH₃ |
| 220 | 3-Br, 4-CH₃ |
| 221 | 2-Cl, 4,5-(CH₃)₂ |
| 222 | 2-Br, 4,5-(CH₃)₂ |
| 223 | 2-Cl, 3,5-(CH₃)₂ |
| 224 | 2-Br, 3,5-(CH₃)₂ |
| 225 | 2,6-Cl₂, 4-CH₃ |
| 226 | 2,6-F₂, 4-CH₃ |
| 227 | 2,6-Br₂, 4-CH₃ |
| 228 | 2,4-Br₂, 6-CH₃ |
| 229 | 2,4-F₂, 6-CH₃ |
| 230 | 2,4-Br₂, 6-CH₃ |
| 231 | 2,6-(CH₃)₂, 4-F |
| 232 | 2,6-(CH₃)₂, 4-Cl |
| 233 | 2,6-(CH₃)₂, 4-Br |
| 234 | 3,5-(CH₃)₂, 4-F |
| 235 | 3,5-(CH₃)₂, 4-Cl |
| 236 | 3,5-(CH₃)₂, 4-Br |
| 237 | 2-CF₃ |
| 238 | 3-CF₃ |
| 239 | 4-CF₃ |
| 240 | 2-OCF₃ |
| 241 | 3-OCF₃ |
| 242 | 4-OCF₃ |
| 243 | 3-OCH₂CHF₂ |
| 247 | 2-CN |
| 248 | 3-CN |
| 249 | 4-CN |
| 25251 | 2-CH₃, 3-Cl |
| 252 | 2-CH₃, 4-Cl |
| 253 | 2-CH₃, 5-C1 |
| 254 | 2-CH₃, 6-Cl |
| 255 | 2-CH₃, 3-F |
| 256 | 2-CH₃, 4-F |
| 257 | 2-CH₃, 5-F |
| 257 | 2-CH₃, 6-F |
| 258 | 2-CH₃, 3-Br |
| 259 | 2-CH₃, 4-Br |
| 260 | 2,5-F₂ |
| 261 | 2,6-F₂ |
| 262 | 3,4-F₂ |
| 263 | 3,5-F₂ |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Reliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz z.B. zum Schutz von Holz, Papier und Textilien eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-a-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls:
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid,
N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis- (1-(2,2,2-trichlor-ethyl) -formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl) -2-methylpropyl] -cis-2, 6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, *α-*(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetylD, L-2-aminobutyrolacton, DL-N-(2,6-Dimethylphenyl)-N-(phenylacetyl)-alaninmethylester, 5-Methyl-5-vinyl-3-(3,5-dichlorphenyl) -2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.
1. N-Methoxy-N-(2-((1-phenyltriazolyl-3)-acetiminoxymethyl)-phenyl) -carbaminsäuremethylester 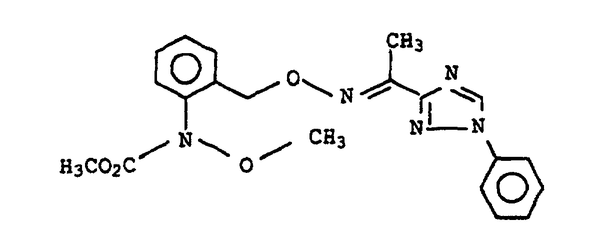
   a) 1-Phenyl-3-acetyltriazol
      Eine Mischung von 2 g (11,3 mmol) Brenztraubensäureamid-1-phenylhydrazon (DE 27 50 813) und 20 ml Triethylorthoformiat wird 1 Stunde auf 100°C erhitzt. Anschließend wird die Reaktionsmischung am Rotationsverdampfer eingeengt und der verbleibende Rückstand wird mit Methyl-t-butylether ausgerührt und abgesaugt. Man erhält 1,9 g (90 %) der Titelverbindung als farblosen Festkörper.
      ¹H-NMR (DMSO-d₆; δ in ppm):
      9,5 (s, 1H, Triazolyl); 7,95 (d, 2H, Phenyl); 7,6 (m, 2H, Phenyl); 7,5 (m, 1H, Phenyl); 2,65 (s, 3H, CH₃)
   b) N-Methoxy-N-(2-((1-phenyltriazolyl-3)-acetiminoxymethyl)-phenyl)-carbaminsäuremethylester
      Eine Mischung von 1,7 g (9,1 mmol) 1-Phenyl-3-acetyltriazol (Beispiel la), 2 g (8,8 mmol) N-Methoxy-N-(2-Aminoxymethyl-phenyl)-carbaminsäuremethylester und einer Spatelspitze p-Toluolsulfonsäure in 30 ml Tetrahydrofuran wird ca. 2 Stunden bei 60°C gerührt. Anschließend verdünnt man die Reaktionsmischung mit Wasser und extrahiert die wäßrige Phase dreimal mit Methylenchlorid. Die vereinigten organischen Phasen werden eingeengt und säulenchromatographisch mit Cyclohexan/Essigester-Gemischen gereinigt. Man erhält 1,0 g des unpolaren Isomeren (verunreinigt) und 2,6 g des polaren Isomeren der Titelverbindung. Nach Ausrühren, jeweils mit Hexan, erhält man 0,2 g (6 %) des unpolaren Isomeren (in der Mutterlauge) als gelbes Öl und 2,4 g (69 %) des polaren Isomeren der Titelverbindung als farblosen Festkörper (Fp = 118°C).
      unpolares Isomeres, ¹H-NMR (DMSO-d₆; δ in ppm):
      8,65 (s, 1H, Triazolyl); 7,3-7,8 (m, 9H, Phenyl); 5,35 (s, 2H, OCH₂); 3,8 (s, 3H, OCH₃); 3,75 (s, 3H, OCH₃); 2,4 (s, 3H, CH₃)
      polares Isomeres, ¹H-NMR (DMSO-d₆; δ in ppm):
      8,55 (s, 1H, Triazolyl); 7,7 (d, 2H, Phenyl); 7,6 (m, 1H, Phenyl); 7,5 (t, 2H, Phenyl); 7,3-7,45 (m, 4H, Phenyl); 5,45 (s, 2H, OCH₂) ; 3,8 (s, 3H, OCH₃); 3,75 (s, 3H, OCH₃); 2,45 (s, 3H, CH₃)
2. M-Methoxy-N-(2-((1-phenyl-5-methoxytriazolyl-3)-acetiminoxymethyl)-phenyl)-carbaminsäuremethylester 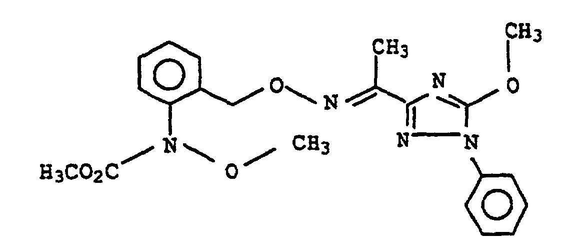
   a) 1-Phenyl-3-acetyl-5-methoxytriazol
      Eine Mischung von 5 g (28 mmol) Brenztraubensäureamid-1-phenylhydrazon (DE 27 50 813), 20 ml Tetramethoxymethan und einer Spatelspitze p-Toluolsulfonsäure wird ca. 3 Stunden auf 120°C erhitzt. Dabei werden die leichtflüchtigen Verbindungen abdestilliert. Anschließend wird die Reaktionsmischung i. Vak. eingeengt und der Rückstand wird säulenchromatographisch mit Cyclohexan/Essigester-Gemischen gereinigt. Das so erhaltene Produkt kristallisiert und wird mit Hexan ausgerührt. Man erhält 3,1 g (52 %) der Titelverbindung als farblosen Festkörper.
      ¹H-NMR (DMSO-d₆; δ in ppm) :
      7,75 (d, 2H, Phenyl); 7,6 (2H, Phenyl); 7,45 (t, 1H, Phenyl); 4,2 (s, 3H, OCH₃) ; 2,55 (s, 3H, CH₃)
   b) 1-Phenyl-3-acetyl-5-methoxytriazol-oxim
      Eine Mischung von 3,1 g (14 mmol) 1-Phenyl-3-acetyl-5-methoxytriazol (Beispiel 2a) und 1,2 g (18 mmol) Hydroxylamin-hydrochlorid in 45 ml Ethanol/Wasser 4:1 wird 3 Tage bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden einmal mit Wasser extrahiert, über MgSO₄ getrocknet und eingeengt. Als Rückstand erhält man 2,2 g (68 %) der Titelverbindung als farblosen Festkörper.
      ¹H-NMR (DMSO-d₆, δ in ppm):
      11,65 (s, 1H, OH); 7,85 (d, 2H, Phenyl); 7,7 (t, 2H, Phenyl); 7,55 (t, 1H, Phenyl); 4,3 (s, 3H, OCE₃); 2,3 (s, 3H, CH₃)
   c) N-Methoxy-N-(2-((1-phenyl-5-methoxytriazolyl-3)-acetiminoxymethyl) -phenyl) -carbaminsäuremethylester
      Eine Mischung von 1,5 g (6,5 mmol) 1-Phenyl-3-acetyl-5-methoxytriazol-oxim (Beispiel 2b) und 0,2 g (8 mmol) Natriumhydrid in 30 ml Dimethylformamid wird gerührt bis die Gasentwicklung beendet ist. Anschließend gibt man 2,5 g (Reinheit ca. 70 %, 7 mmol) N-Methoxy-N-(2-brommethylphenyl)-carbaminsäuremethylester (WO 93/15046) hinzu und rührt ca. 1 Stunde bei Raumtemperatur. Anschließend verdünnt man die Reaktionsmischung mit Wasser und extrahiert die wäßrige Phase dreimal mit Methyl-t-butylether. Die vereinigten organischen Phasen werden einmal mit Wasser extrahiert, über MgSO₄ getrocknet und eingeengt. Der Rückstand wird säulenchromatographisch mit Cyclohexan/Essigester-Gemische gereinigt. Das Produkt kristallisiert und wird mit Methyl-t-butylether/ Hexan ausgerührt. Man erhält 1 g (36 %) der Titelverbindung als farblosen Festkörper (Fp = 107°C).
      ¹H-NMR (CDCl₃; δ in ppm):
      7,75 (d, 2H, Phenyl); 7,6 (s, breit, 1H, Phenyl); 7,2-7,55 (m, 6H, Phenyl); 5,4 (s, 2H, OCH₂); 4,25 (s, 3H, OCH₃); 3,75 (2s, je 3H, 2xOCH₃); 2,35 (s, 3H, CH₃)
3. N-Methoxy-N-(2-((1-phenyl-5-trifluormethyltriazolyl-3)-acetiminoxymethyl)-phenyl)-carbaminsäuremethylester 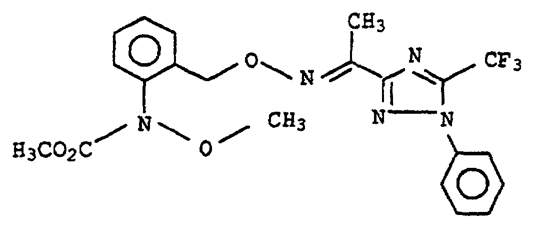
   a) 1-Phenyl-3-acetyl-5-trifluormethyltriazol
      Eine Mischung von 3,5 g (20 mmol) Brenztraubensäureamid-1-phenylhydrazon (DE 27 50 813), 2,5 g (30 mol) Pyridin und 5,2 g (25 mmol) Trifluoracetanhydrid in 30 ml Methylenchlorid wird ca. 1,5 Stunden bei Raumtemperatur gerührt. Anschließend wird die Reaktionsmischung mit Wasser und verdünnter Salzsäure gewaschen, über MgSO₄ getrocknet und eingeengt. Der Rückstand wird mit Cyclohexan/Essigester-Gemischen über eine kurze Kieselgelsäule chromatographiert. Das Produkt kristallisiert beim Eindampfen aus und wird mit Methyl-t-butylether/Hexan ausgerührt. Man erhält 3,5 g (69 %) der Titelverbindung als farblosen Festkörper.
      ¹H-NMR (CDCl₃; δ in ppm) :
      7,45-7,7 (m, 5H, Phenyl); 2,75 (s, 3H, CH₃)
   b) 1-Phenyl-3-acetyl-5-trifluormethyltriazol-oxim
      Eine Mischung von 3,5 g (14 mmol) 1-Phenyl-3-acetyl-5-trifluormethyltriazol (Beispiel 3a) und 1,2 g (18 mmol) Hydroxylamin-hydrochlorid in 50 ml Ethanol/Wasser 4:1 wird 3 Tage bei Raumtemperatur gerührt. Anschließend wird die Reaktionsmischung i.Vak. eingeengt. Der Rückstand wird mit Wasser versetzt und die wäßrige Phase wird mit Methylenchlorid extrahiert. Die vereinigten organischen Phasen werden mit Wasser extrahiert über MgSO₄ getrocknet und eingeengt. Als Rückstand erhält man 2,7 g (71 %) der Titelverbindung als farblosen Festkörper.
      ¹H-NMR (CDCl₃; δ in ppm):
      11,8 (s, 1H, OH); 7,6 (s, 5H, Phenyl); 2,2 (s, 3H, CH₃)
   c) N-Methoxy-N-(2-((1-phenyl-5-trifluormethyltriazol-3)-acetiminoxymethyl)-phenyl) -carbaminsäuremethylester
      Eine Mischung von 1,5 g (5,6 mmol) 1-Phenyl-3-acetyl-5-trifluormethyltriazol-oxim (Beispiel 3b) und 0,17 g (7 mmol) Natriumhydrid in 30 ml Dimethylformamid wird gerührt bis die Gasentwicklung beendet ist. Anschließend gibt man 2,1 g (Reinheit ca. 70 %, 5,6 mmol) N-Methoxy-N- (2-brommethylphenyl)-carbaminsäuremethylester (WO 93/15046) hinzu und rührt ca. 2 Stunden bei Raumtemperatur. Anschließend verdünnt man die Reaktionsmischung mit Wasser und extrahiert die wäßrige Phase dreimal mit Methyl-t-butylether. Die vereinigten organischen Phasen werden über MgSO₄ getrocknet und eingeengt. Der Rückstand wird säulenchromatographisch mit Cyclohexan/Essigester-Gemischen gereinigt. Man erhält 1,5 g (58 %) der Titelverbindung.
      ¹H-NMR (CDCl₃; δ in ppm) :
      7,45-7,6 (m, 6H, Phenyl); 7,35 (m, 3H, Phenyl); 3,8 (s, 3H, OCH₃); 3,75 (s, 3H, OCH₃); 2,4 (s, 3H, CH₃)

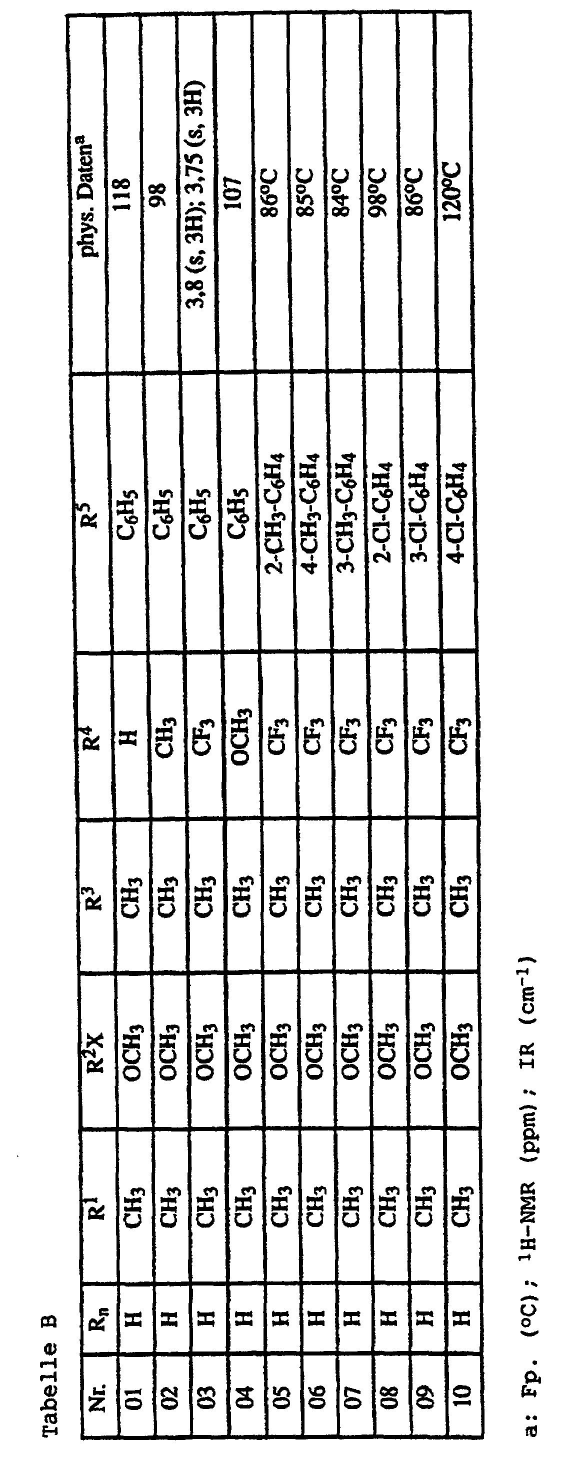

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 250 ppm). Nach 8 Tagen wurden die Pflanzen mit einer Zoosporenaufschwemmung des Pilzes Plasmopara *viticola* besprüht und 5 Tage bei 20-30°C bei hoher Luftfeuchtigkeit aufbewahrt. Vor der Beurteilung wurden die Pflanzen danach für 16 h bei hoher Luftfeuchtigkeit aufbewahrt. Die Auswertung erfolgte visuell.

In diesem Test zeigten die mit den erfindungsgemäßen Verbindungen 1, 2, 3, 5, 6, 7 und 8 behandelten Pflanzen einen Befall von 15 % der Blattflächen und weniger, während die unbehandelten (Kontroll-) Pflanzen zu 70 % befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Iminooxymethylenanilide der Formel I 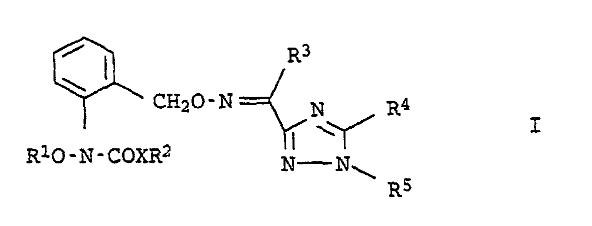 in der der Index und die Substituenten die folgende Bedeutung haben:
X O;
R¹ Wasserstoff, C₁-C₄-Alkyl, Allyl, Propargyl oder Methoxymethyl;
R² C₁-C₄-Alkyl, Cyclopropyl oder Trifluormethyl;
R³ Cyano, Halogen, C₁-C₄-Alkyl, Trifluormethyl, Cyclopropyl, Methoxy, Ethoxy und Methylthio;
R⁴ Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio;
Phenyl oder Hetaryl, wobei diese Reste einen bis drei der folgenden Gruppen tragen kann: Cyano, Halogen, C₁-C₄-Alkyl und CRⁱⁱⁱ=NOR^{iv};
R⁵ Phenyl, welches ein bis drei der folgenden Reste tragen kann: Cyano, Halogen, C₁-C₄-Alkyl oder CRⁱⁱⁱ=NOR^{iv}; oder welches durch C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₂-C₆-Alkenyloxy, C₂-C₆-Alkinyloxy, C₁-C₆-Halogenalkyloxy, C₂-C₆-Halogenalkenyloxy, C₂-C₆-Halogenalkinyloxy, Phenyl, Phenoxy, Hetaryl oder Hetaryloxy substituiert ist, wobei die Phenyl- und Hetaryl-Gruppen ihrerseits durch Cyano, Halogen, C₁-C₄-Alkyl und CRⁱⁱⁱ=NOR^{iv} substituiert sein können;
Rⁱⁱⁱ Wasserstoff, C₁-C₆-Alkyl, C₂-C₆-Alkenyl oder C₂-C₆-Alkinyl;
R^{iv} C₁-C₆-Alkyl, C₂-C₆-Alkenyl, C₂-C₆-Alkinyl oder Phenyl-C₁-C₆-alkyl;
Hetaryl 5-Ring Heteroaromat enthaltend ein bis drei Stickstoffatome und/oder ein Sauerstoff- oder Schwefelatom; oder 6-Ring Heteroaromat enthaltend ein bis vier Stickstoffatome.

2. Verbindungen der Formel I gemäß Anspruch 1, in denen R¹ für C₁-C₄-Alkyl steht.

3. Verbindungen der Formel I gemäß Anspruch 1, in denen R² für C₁-C₄-Alkyl steht.

4. Verbindungen der Formel I gemäß Anspruch 1, in denen R³ für C₁-C₄-Alkyl steht.

5. Verbindungen der Formel I gemäß Anspruch 1, in denen R⁴ für Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio steht.

6. Verbindungen der Formel I gemäß Anspruch 1, in denen R⁵ für Phenyl steht, welches ein bis drei der folgenden Reste tragen kann: Cyano, Halogen, C₁-C₄-Alkyl oder CRⁱⁱⁱ=NOR^{1v}.

7. Verfahren zur Herstellung der Verbindungen der Formel I gemäß Anspruch 1, in denen R¹ nicht Wasserstoff bedeutet, **dadurch gekennzeichnet**, daß man ein Methylenanilid der Formel II 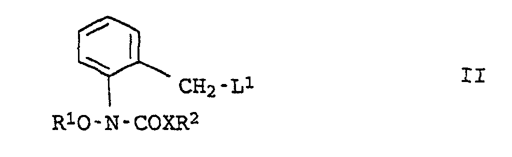 in der L¹ für eine Abgangsgruppe steht, mit einem Oxim der Formel III 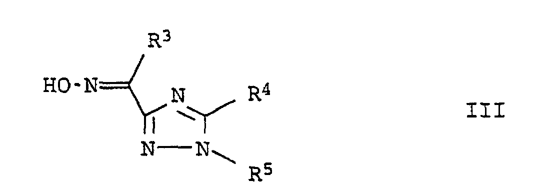 oder dessen Salz umsetzt.

8. Verfahren zur Herstellung der Verbindungen der Formel I gemäß Anspruch 1, **dadurch gekennzeichnet**, daß man einen Hydroxylaminether der Formel IV 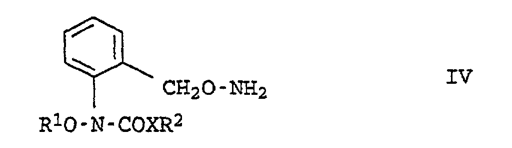 in einem inerten organischen Lösungsmittel mit einem Triazolylketon der Formel V 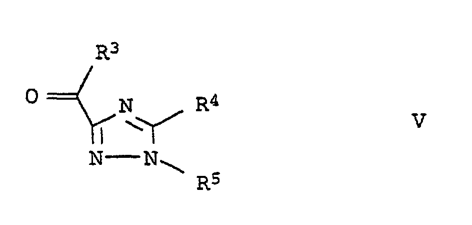 umsetzt.

9. Verbindungen der Formel IX 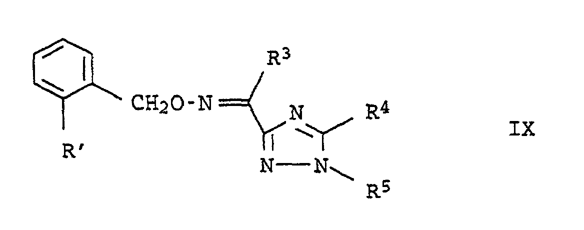 in denen R³ bis R⁵ die in Anspruch 1 gegebene Bedeutung haben und R' für NO₂, NHOH oder N(OR¹)-CO₂C₆H₅ steht.

10. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

11. Verwendung der Verbindungen I gemäß Anspruch 1 zur Herstellung eines zur Bekämpfung von Schädlingen oder Schadpilzen geeigneten Mittels.

12. Verfahren zur Bekämpfung von Schadpilzen, **dadurch gekennzeichnet**, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

13. Verfahren zur Bekämpfung von Schädlingen, **dadurch gekennzeichnet**, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. An iminooxymethyleneanilide of the formula I 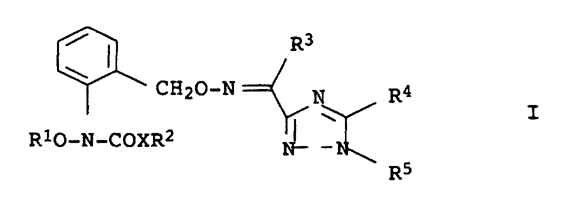 where the index and the substituents have the following meanings:
x is O;
R¹ is hydrogen, C₁-C₄-alkyl, allyl, propargyl or methoxymethyl;
R² is C₁-C₄-alkyl, cyclopropyl or trifluoromethyl;
R³ is cyano, halogen, C₁-C₄-alkyl, trifluoromethyl, cyclopropyl, methoxy, ethoxy or methylthio;
R⁴ is hydrogen, cyano, halogen, C₁-C₄-alkyl, C₁-C₄-haloalkyl, C₁-C₄-alkoxy, C₁-C₄-haloalkoxy or C₁-C₄-alkylthio;
phenyl or hetaryl, where these radicals can carry one to three of the following groups: cyano, halogen, C₁-C₄-alkyl and CRⁱⁱⁱ=NOR^{iv};
R⁵ is phenyl which can carry one to three of the following radicals: cyano, halogen, C₁-C₄-alkyl or CRⁱⁱⁱ=NOR^{iv}; or which is substituted by C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₆-alkoxy, C₂-C₆-alkenyloxy, C₂-C₆-alkynyloxy, C₁-C₆-haloalkyloxy, C₂-C₆-haloalkenyloxy, C₂-C₆-haloalkynyloxy, phenyl, phenoxy,hetaryl or hetaryloxy, where the phenyl and hetaryl groups, for their part, can be substituted by cyano, halogen, C₁-C₄-alkyl and CRⁱⁱⁱ=NOR^{iv};
Rⁱⁱⁱ is hydrogen, C₁-C₆-alkyl, C₂-C₆-alkenyl or C₂-C₆-alkynyl;
R^{iv} is C₁-C₆-alkyl, C₂-C₆-alkenyl, C₂-C₆-alkynyl or phenyl-C₁-C₆-alkyl;
Hetaryl is a 5-ring heteroaromatic comprising one to three nitrogen atoms and/or one oxygen or sulfur atom, or is a 6-ring heteroaromatic comprising one to four nitrogen atoms.

2. A compound of the formula I as claimed in claim 1, in which R¹ is C₁-C₄-alkyl.

3. A compound of the formula I as claimed in claim 1, in which R² is C₁-C₄-alkyl.

4. A compound of the formula I as claimed in claim 1, in which R³ is C₁-C₄-alkyl.

5. A compound of the formula I as claimed in claim 1, in which R⁴ is hydrogen, cyano, halogen, C₁-C₄-alkyl, C₁-C₄-haloalkyl, C₁-C₄-alkoxy, C₁-C₄-haloalkoxy or C₁-C₄-alkylthio.

6. A compound of the formula I as claimed in claim 1, in which R⁵ is phenyl which can carry one to three of the following radicals: cyano, halogen, C₁-C₄-alkyl or CRⁱⁱⁱ=NOR^{iv}.

7. A process for preparing the compounds of the formula I as claimed in claim 1, where R¹ is not hydrogen, which comprises reacting a methyleneanilide of the formula II 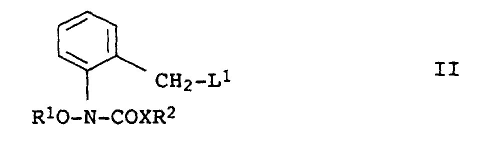 where L¹ is a leaving group, with an oxime of the formula III 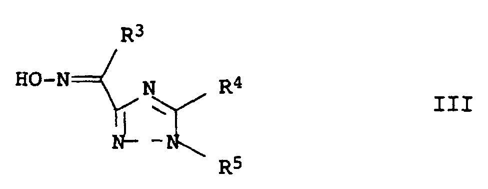 or its salt.

8. A process for preparing the compounds of the formula I as claimed in claim 1, which comprises reacting a hydroxylamine ether of the formula IV 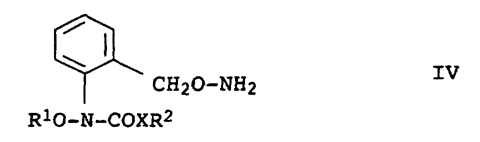 in an inert organic solvent with a triazolyl ketone of the formula V

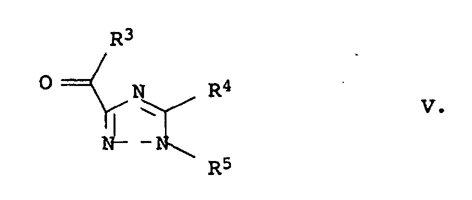

9. A compound of the formula IX 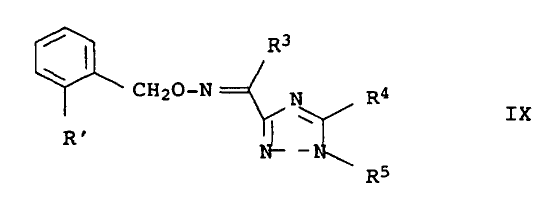 where R³ to R⁵ have the meanings given in claim 1 and R' is NO₂, NHOH or N(OR¹)-CO₂C₆H₅.

10. A composition suitable for controlling pests or harmful fungi, containing a solid or liquid carrier and a compound of the general formula I as claimed in claim 1.

11. The use of the compounds I as claimed in claim 1 for preparing a composition suitable for controlling pests or harmful fungi.

12. A method of controlling harmful fungi,-which comprises treating the fungi or the materials, plants, soil or seed to be protected from fungal attack with an effective amount of a compound of the general formula I as claimed in claim 1.

13. A method of controlling pests, which comprises treating the pests or the materials, plants, soil or seed to be protected from them with an effective amount of a compound of the general formula I as claimed in claim 1.

## Revendications

1. Iminooxyméthylène-anilides de formule I 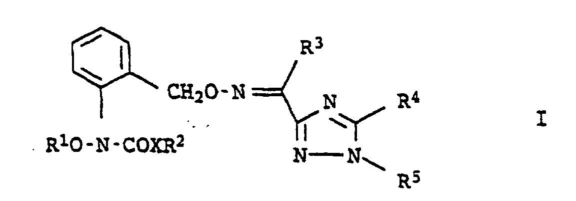 dans laquelle l'indice et les symboles ont les significations suivantes :
X : O ;
R¹ : l'hydrogène, un groupe alkyle en C1-C4, allyle, propargyle ou méthoxyméthyle ;
R² : un groupe alkyle en C1-C4, cyclopropyle ou trifluorométhyle ;
R³ : un groupe cyano, un halogène, un groupe alkyle en C1-C4, trifluorométhyle, cyclopropyle, méthoxy, éthoxy ou méthylthio ;
R⁴ : l'hydrogène, un groupe cyano, un halogène, un groupe alkyle en C1-C4, halogénoalkyle en C1-C4, alcoxy en C1-C4, halogénoalcoxy en C1-C4 ou alkylthio en C1-C4 ; un groupe phényle ou hétéroaryle, ces groupes pouvant porter un à trois des substituants suivants : cyano, halogéno, alkyle en C1-C4 et CRⁱⁱⁱ=NOR^{iv};
R⁵ : un groupe phényle qui peut porter un à trois des substituants suivants : cyano, halogéno, alkyle en C1-C4 ou CRⁱⁱⁱ=NOR^{iv} ; ou qui porte des substituants alkyle en C1-C6, alcényle en C2-C6, alcynyle en C2-C6, halogénoalkyle en C1-C6, halogénoalcényle en C2-C6, halogénoalcynyle en C2-C6, alcoxy en C1-C6, alcényloxy en C2-C6, alcynyloxy en C2-C6, halogénoalcoxy en C1-C6, halogénoalcényloxy en C2-C6, halogénoalcynyloxy en C2-C6, phényle, phénoxy, hétéroaryle ou hétéroaryloxy, les groupes phényle et hétéroaryle pouvant eux-mêmes porter des substituants cyano, halogéno, alkyle en C1-C4 et CRⁱⁱⁱ=NOR^{iv} ;
Rⁱⁱⁱ : l'hydrogène, un groupe alkyle en C1-C6, alcényle en C2-C6 ou alcynyle en C2-C6 ;
R^{iv} : un groupe alkyle en C1-C6, alcényle en C2-C6, alcynyle en C2-C6 ou phényl-alkyle en C1-C6 ;
hétéroaryle : cycle hétéroaromatique à cinq chaînons contenant un à trois atomes d'azote et/ou un atome d'oxygène ou de soufre ; ou bien cycle hétéroaromatique à six chaînons contenant un à quatre atomes d'azote.

2. Composés de formule I selon la revendication 1, dans laquelle R¹ représente un groupe alkyle en C1-C4.

3. Composés de formule I selon la revendication 1, dans laquelle R² représente un groupe alkyle en C1-C4.

4. Composés de formule I selon la revendication 1, dans laquelle R³ représente un groupe alkyle en C1-C4.

5. Composés de formule I selon la revendication 1, dans laquelle R⁴ représente l'hydrogène, un groupe cyano, un halogène, un groupe alkyle en C1-C4, halogénoalkyle en C1-C4, alcoxy en C1-C4, halogénoalcoxy en C1-C4 ou alkylthio en C1-C4.

6. Composés de formule I selon la revendication 1, dans laquelle R⁵ représente un groupe phényle qui peut porter un à trois des substituants suivants : cyano, halogéno, alkyle en C1-C4 ou CRⁱⁱⁱ=NOR^{iv}.

7. Procédé de préparation des composés de formule I de la revendication 1 dans laquelle R¹ a une signification autre que l'hydrogène, caractérisé par le fait que l'on fait réagir un méthylène-anilide de formule II 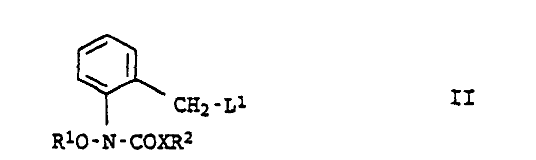 dans laquelle L¹ représente un substituant éliminable, avec une oxime de formule III 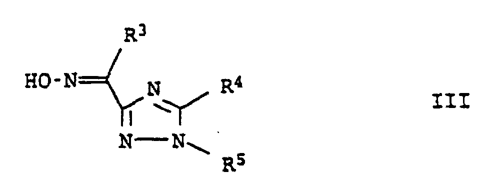 ou l'un de ses sels.

8. Procédé de préparation des composés de formule I de la revendication 1, caractérisé par le fait que l'on fait réagir un éther d'hydroxylamine de formule IV 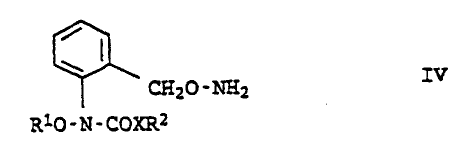 dans un solvant organique inerte avec une triazolylcétone de formule V

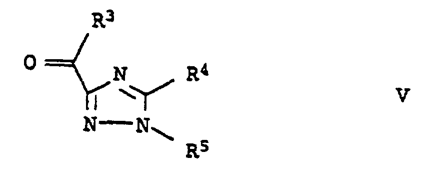

9. Composés de formule IX 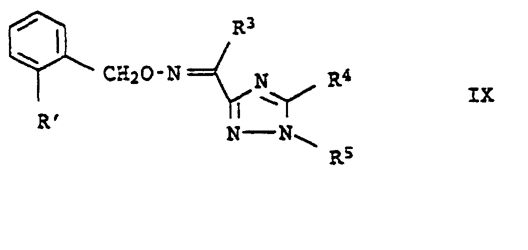 dans laquelle R³ à R⁵ ont les significations indiquées dans la revendication 1 et R' représente NO₂, NHOH ou N(OR¹)-CO₂C₆H₅.

10. Produit approprié à la lutte contre les parasites ou les mycètes nuisibles, contenant un véhicule solide ou liquide et un composé de formule générale I de la revendication 1.

11. Utilisation des composés I de la revendication 1 pour la préparation d'un produit approprié à l'utilisation pour la lutte contre les parasites ou les mycètes nuisibles.

12. Procédé pour combattre les mycètes nuisibles, caractérisé par le fait que l'on traite les mycètes ou les matériaux, végétaux, sols ou semences menacés d'une attaque par les mycètes par une quantité efficace d'un composé de formule générale I de la revendication 1.

13. Procédé pour combattre les parasites, caractérisé par le fait que l'on traite les parasites ou les matériaux, végétaux, sols ou semences menacés d'une attaque par les parasites par une quantité efficace d'un composé de formule générale I de la revendication 1.
